# EUROPEAN PATENT SPECIFICATION

(11) **EP 4 148 018 B1**
(45) Date of publication and mention of the grant of the patent: **24.09.2025**
(21) Application number: 21800964.5
(22) Date of filing: 06.05.2021
(51) Int. Cl.: G21G 1/10, C01B 7/00, A61K 47/54, A61K 51/04, A61P 35/00, C07B 59/00, C07D 319/04, G21G 1/00

(54) **SIMPLE ASTATINE CONCENTRATION METHOD**
EINFACHES VERFAHREN ZUR KONZENTRATION VON ASTATIN
MÉTHODE DE CONCENTRATION D'ASTATE SIMPLE

(30) Priority: 07.05.2020 JP 2020081819
(43) Date of publication of application: 15.03.2023
(73) Proprietor: Institute of Science Tokyo, Tokyo 152-8550 (JP); Fukushima Medical University, Fukushima 960-1295 (JP)
(72) Inventor: TANAKA, Hiroshi, Tokyo 152-8550 (JP); TAKAHASHI, Kazuhiro, Fukushima-shi, Fukushima 960-1295 (JP); SUZUKI, Miho, Fukushima-shi, Fukushima 960-1295 (JP)
(74) Representative: J A Kemp LLP
(86) International application number: PCT/JP2021/017415
(87) International publication number: WO 2021/225147

(56) References cited:
- WO-A1-2019/112034
- JP-A- 2018 534 345
- JP-A- 2018 536 031
- US-A- 5 102 651
- EMMA ANEHEIM ET AL: "Automated astatination of biomolecules - a stepping stone towards multicenter clinical trials", SCIENTIFIC REPORTS, vol. 5, 14 July 2015 (2015-07-14), pages 1 - 11, XP055245286, DOI: 10.1038/srep12025

## Description

### [Technical Field]

The present invention relates to a method for producing astatine-211, and a method for producing an astatine-211-labeled compound using the astatine-211 produced by this method.

### [Background Art]

Astatine is one of the halogen elements and considered to have chemical properties similar to those of iodine. Astatine has multiple isotopes but no stable isotopes, all of which are radioactive isotopes. Astatine-211 is one of the radioactive isotopes and has recently attracted attention since it can be used for RI internal therapy that selectively destroys cancer cells.

As a production method of astatine-211, a wet separation method and a dry separation method are known. Since the wet separation method has problems such as contamination with impurities, the dry separation method is currently the mainstream. In the dry separation method, 1) a step of generating astatine-211 by irradiating bismuth with α rays, 2) a step of heating the generated astatine-211 to vaporize, 3) a step of cooling the astatine-211 that has been vaporized to collect the astatine-211 with a solvent, and 4) a step of removing the solvent are sequentially performed, to produce astatine-211. Although it has long been pointed out that astatine scatters together with the solvent when removing the solvent since astatine is easily volatile, scattering of astatine-211 can be prevented by using chloroform as a solvent (Non Patent Literature 1). An automated astatination of biomolecules is known from Non Patent Literature 2.

### [Citation List]

### [Non Patent Literature]

[Non Patent Literature 1] E. Aneheim et al., Sci Rep. 2019 Nov 4; 9 (1): 15900
[Non Patent Literature 2] E. Aneheim et al., Sci Rep. 2015 Jul 14; 5: 12025

### [Summary of Invention]

### [Technical Problem]

As described above, use of chloroform as a solvent can prevent scattering of astatine-211 and allow collection of astatine-211 with high recovery. However, when chloroform is used as a solvent, astatine-211 is contaminated, albeit in very small amounts, with chloride ions liberated from chloroform. Such contamination with chloride ions does not matter much when the labeling reaction with astatine-211 is an electrophilic substitution reaction. However, when the labeling reaction is a nucleophilic substitution reaction, the chloride ions react with a labeling precursor instead of astatine-211, which is a big problem.

The present invention has accomplished under such a situation, and it is an object of the present invention to provide a method for preventing astatine-211 from scattering without using chloroform when removing a solvent from an astatine-211 solution.

### [Solution to Problem]

As a result of diligent studies in order to solve the above problem, the present inventors have found that scattering of astatine-211 can be prevented without using chloroform by collecting astatine-211 with a chlorine-free solvent such as alcohol, adding a carbonate or bicarbonate to the astatine solution obtained, and then removing the solvent, thereby having accomplished the present invention.

The present invention is defined by the appended claims.

Japanese Patent Application No. 2020-081819, is the basis of the priority of the present application.

### [Advantageous Effect of Invention]

The present invention provides a novel method for producing astatine-211. According to the production method of the present invention, astatine-211 without contamination of chloride ions can be produced in high yield.

### [Description of Embodiments]

Hereinafter, the present invention will be described in detail.

### (A) Method for producing astatine-211

The method for producing astatine-211 of the present invention comprises the following steps (1) to (5).

In step (1), bismuth is irradiated with α rays to generate astatine-211.

Bismuth can be irradiated with α rays using an accelerator. Irradiation with α rays causes a nuclear reaction of ²⁰⁹Bi(α,2n)²¹¹At to generate astatine-211.

In step (2), the astatine-211 generated in step (1) is heated to vaporize.

The astatine-211 is heated in a furnace. The temperature in the furnace is not specifically limited, as long as it is a temperature at which the astatine-211 can be vaporized, but is preferably 600 to 900°C, more preferably 800 to 900°C.

In step (3), the astatine-211 that has been vaporized in step (2) is cooled and collected with a volatile and polar solvent to obtain an astatine-211 solution.

The method for cooling the astatine-211 is not specifically limited, and examples thereof can include a method of feeding the astatine-211 that has been vaporized in a furnace into a tube (cooling trap) set in a tank filled with a low-temperature liquid using a carrier gas. The carrier gas may be those generally used in a dry separation method of astatine-211 such as helium or a mixed gas of nitrogen and oxygen. The liquid used for the cooling trap also may be those generally used in a dry separation method of astatine-211, and examples thereof may include liquid nitrogen and ethanol with dry ice.

The method for collecting astatine-211 with a solvent is not specifically limited, and in the case of cooling the astatine-211 using a cooling trap, as described above, examples thereof can include a method of passing the solvent through the tube of the cooling trap.

The solvent to be used is not specifically limited, as long as it is volatile and polar. For example, methanol, ethanol, 2-propanol, or acetonitrile can be used. Among these, methanol is preferable since it has a low boiling point and is easily volatile.

The solvent is preferably free from water. This is because the labeling reaction of astatine-211 may be performed under anhydrous conditions (for example, the labeling reaction of astatine-211 of formula (I)-1 or formula (I)-2 described below is performed under anhydrous conditions), and water is difficult to volatilize and may remain to adversely affect the reaction.

Further, the solvent is preferably free from halogen atoms in its molecule. This is because, in the case of using a solvent containing a halogen atom in its molecule, halide ions liberated from the solvent may adversely affect the reaction between astatine-211 and a labeling precursor compound.

In step (4), a weak acid salt is added to the astatine-211 solution obtained in step (3) to obtain an astatine-211 solution containing the weak acid salt.

As a weak acid salt, a carbonate, a bicarbonate, a phosphate, a hydrogen phosphate, a dihydrogen phosphate, an acetate, or the like can be used. Among these, a carbonate or bicarbonate is preferably used. This is because carbonate ions and bicarbonate ions contained in the carbonate and bicarbonate vaporize as CO₂ and H₂O, and thus can be easily removed. The carbonate and bicarbonate are preferably salts of alkali metals, and preferred examples thereof include NaHCO₃, Na₂CO₃, KHCO₃, K₂CO₃, CsHCO₃, and Cs₂CO₃.

The concentration of the weak acid salt in the astatine-211 solution is not specifically limited, as long as it can prevent scattering of astatine-211, but is preferably 0.2 to 2.0 M, more preferably 0.3 to 1.5 M, even more preferably 0.5 to 1.0 M.

In step (5), the solvent is removed from the astatine-211 solution containing the weak acid salt obtained in step (4).

The method for removing the solvent is preferably a method in which astatine-211 is difficult to scatter, and examples thereof can include a method for removing a solvent by spraying a nitrogen gas.

### (B) First production method of astatine-211-labeled compound

The first production method of an astatine-211-labeled compound of the present invention is a method for producing an astatine-211-labeled compound comprising reacting the astatine-211 produced by the method above for producing astatine-211 of the present invention (which will be hereinafter referred to as "the astatine-211 of the present invention") with a labeling precursor compound to produce an astatine-211-labeled compound, wherein the reaction is a nucleophilic substitution reaction.

The astatine-211 collected with a solvent such as alcohol is presumed to be present in the anionic state (²¹¹At⁻) in the astatine-211 solution. Accordingly, the astatine-211 of the present invention acts as a nucleophilic reagent in a nucleophilic substitution reaction and reacts with various labeling precursor compounds to generate an astatine-211-labeled compound.

The labeling precursor compound to be used is not specifically limited, but a compound having a leaving group for a nucleophilic substitution reaction of astatine-211 is preferable. The leaving group is not specifically limited, and examples thereof include alkylsulfonyloxy groups such as a methanesulfonyloxy group, haloalkylsulfonyloxy groups such as a trifluoromethanesulfonyloxy group, or arylsulfonyloxy groups such as a p-toluenesulfonyloxy group. Suitable examples of the leaving group can include a trifluoromethanesulfonyloxy group or a p-toluenesulfonyloxy group. Further, a compound that stabilizes astatine-211 in vivo has been reported recently (WO2019/151384). The labeling precursor compound to be used in the present invention may be those that generate such a compound by reaction with astatine-211. For example, a compound represented by formula (I)-1 or formula (I)-2 below generates an astatine-211-containing compound (compound represented by formula (II) described below) that is stable in vivo by reaction with astatine-211, and such a compound may be used as a labeling precursor compound.

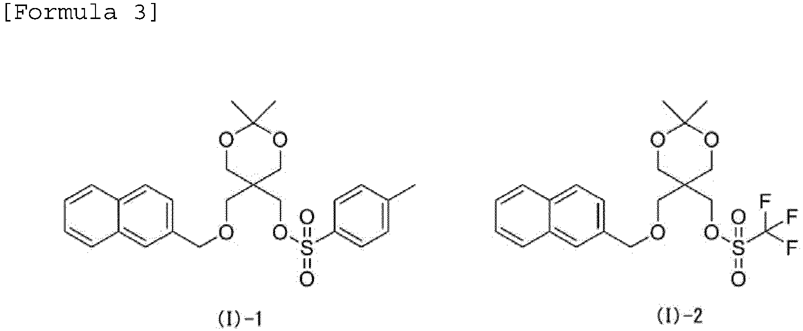

In the case of using a compound having a p-toluenesulfonyloxy group or trifluoromethanesulfonyloxy group as a labeling precursor compound, the astatine-211-labeled compound to be generated is a compound in which the p-toluenesulfonyloxy group or trifluoromethanesulfonyloxy group in the labeling precursor compound is substituted with astatine-211. Further, in the case of using the compound represented by formula (I)-1 or formula (I)-2 above as a labeling precursor compound, the astatine-211-labeled compound to be generated is a compound represented by formula (II) below.

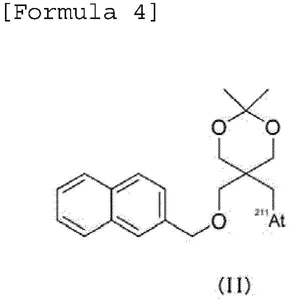

The temperature, the time, and the solvent to be used in the reaction of the astatine-211 of the present invention with the labeling precursor compound can be determined according to each reaction. For example, in the case of a reaction of the astatine-211 of the present invention with the compound represented by formula (I)-1, the reaction temperature can be 50 to 150°C, preferably 80 to 120°C, the reaction time can be 10 to 60 minutes, preferably 20 to 40 minutes, and acetonitrile can be used as a solvent. Further, in the case of a reaction of the astatine-211 of the present invention with the compound represented by formula (I)-2, the reaction temperature can be 10 to 30°C, preferably 15 to 25°C, the reaction time can be 10 to 60 minutes, preferably 20 to 40 minutes, and acetonitrile can be used as a solvent.

### (C) Second production method of astatine-211-labeled compound

The second production method of the astatine-211-labeled compound of the present invention is a method for producing an astatine-211-labeled compound comprising reacting the astatine-211 of the present invention with a labeling precursor compound to produce an astatine-211-labeled compound, wherein the reaction is an electrophilic substitution reaction.

The electrophilic substitution reaction is not specifically limited but is preferably an aromatic electrophilic substitution reaction in which a leaving group on an aromatic ring is substituted with astatine-211.

The labeling precursor compound to be used is not specifically limited, but a compound having a leaving group that can be easily substituted with astatine-211, such as a trialkylsilyl group and a trialkylstannyl group, is preferable. Examples of the trialkylsilyl group can include a trimethylsilyl group, a triethylsilyl group, a tri n-propylsilyl group, a triisopropylsilyl group, and a tri n-butylsilyl group. Among these, a trimethylsilyl group (-SiMe₃) is preferable. Examples of the trialkylstannyl group can include a trimethylstannyl group, a triethylstannyl group, a tripropylstannyl group, and a tributylstannyl group. Among these, a tributylstannyl group (-SnBu₃) is preferable. In addition, since astatine-211-metaastatobenzylguanidine has recently attracted attention as an α-ray cancer therapeutic agent, metatrimethylsilylbenzylguanidine that is the precursor of this compound may be used as a labeling precursor compound.

### EXAMPLES

Hereinafter, the present invention will be described further in detail by way of examples, but the present invention is not limited to these examples.

### [Example 1] Measurement of ²¹¹At residual rate

Astatine-211 (²¹¹At) was produced by irradiating a stable isotope of bismuth with an α beam using a cyclotron to cause a nuclear reaction of ²⁰⁹Bi(α,2n)²¹¹At. The ²¹¹At was isolated from bismuth after irradiation (using a mixed gas of nitrogen and oxygen as a carrier gas) by heating in a furnace at 850°C for distillation and was collected by washing a trap tube on the downstream in any solvent. The ²¹¹At solution thus obtained was concentrated by the following method.

100 µL of the ²¹¹At solution collected with methanol was added to a vial into which 72.4 µmol of a bicarbonate salt and a carbonate salt of sodium, potassium, cesium, and the like was put in advance, and the resulting mixture was mixed well. The ²¹¹At solution was mixed with the bicarbonate or carbonate within 60 minutes after collection. Thereafter, a vent needle connected to activated carbon was attached thereto, and the solvent was removed with a nitrogen gas. The radioactivity of ²¹¹At in the vial before and after the solvent was removed was measured, to determine the ²¹¹At residual rate (Table 1).

**[Table 1]**

| Type of salt | ²¹¹At residual rate | Number of trials |
|---|---|---|
| No salt | 37% ± 25% | 2 |
| NaHCO₃ | 98% ± 2% | 5 |
| Na₂CO₃ | 95% ± 1% | 4 |
| KHCO₃ | 97% ± 1% | 2 |
| K₂CO₃ | 98% ± 2% | 29 |
| CsHCO₃ | 97% ± 1% | 2 |
| Cs₂CO₃ | 99% ± 1% | 2 |

As shown in Table 1, the ²¹¹At residual rate significantly increased when any salt was added, as compared with the case where no salt was added.

### [Example 2] Measurement of generation rate of ²¹¹At-labeled compound

After removing the solvent from the ²¹¹At solution, a labeling precursor (I)-1 or (I)-2 (21.3 µmol/300 µL) dissolved in anhydrous acetonitrile was added and mixed using a vortex mixer. The labeling precursor (I)-1 was reacted at 100°C, and the labeling precursor (I)-2 was reacted at room temperature for 30 minutes, to determine the generation rate of the ²¹¹At-labeled compound by TLC (Table 2). Thin-layer chromatography was performed on a 0.2-mm E. Merck silica gel plate (60F-254) and visualized by the imaging plate (IP) system, and ROI was set to the spot (Rf 0.4) of the labeled body (II) for calculation. The developing solvent used was hexane:ethyl acetate = 4:1, the IP used was BAS IP SR 2025 E, the exposure time was 2 hours, the IP reader was CD 35 Bio (Elysia-Raytest, Straubenhardt, Germany), and the analysis software was Aida Image Analyzer v.5.1 (Elysia-Raytest, Straubenhardt, Germany).

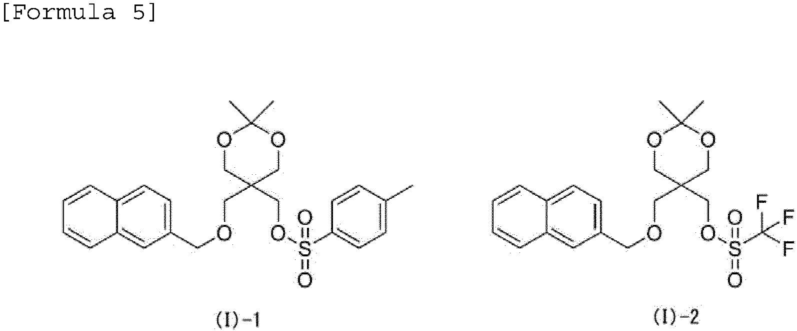

**[Table 2]**

| Type of salt | Precursor (I)-1 (number of trials) | Precursor (1)-2 (number of trials) |
|---|---|---|
| NaHCO₃ | 22% ± 2% (n = 2) | 11% (n = 1) |
| Na₂CO₃ | 25% ± 2% (n = 2) | 19% (n = 1) |
| KHCO₃ | 34% (n = 1) | 41% (n = 1) |
| K₂CO₃ | 32% ± 10% (n = 5) | 85% (n = 1) |
| CsHCO₃ | 48% (n = 1) | 60% (n = 1) |

As shown in Table 2, there was a difference in generation rate depending on the type of the salt used. The generation rate of the labeling precursor (I)-1 was high when using CsHCO₃, and the generation rate of the labeling precursor (I)-2 was high when using K₂CO₃.

### [Example 3] Synthesis of labeling precursor and ²¹¹At-labeled compound analogue (iodine)

The labeling precursor (I)-1 ((2,2-dimethyl-5-((naphthalene-2-ylmethoxy)methyl)-1,3-dioxane-5-yl)methyl(4-methyl)benzene sulfonate) and the labeling precursor (I)-2 (2,2-dimethyl-5-((naphthalene-2-ylmethoxy)methyl)-1,3-dioxane-5-yl)methyl trifluoromethanesulfonate) were synthesized. Further, in order to show synthesis of the ²¹¹At-labeled compound (I)-2 is possible, a compound labeled with iodine having similar chemical properties (5-iodomethyl-5-((naphthalene-2-ylmethoxy)methyl)-2,2,-dimethyl-1,3-dioxane) instead of ²¹¹At was synthesized.

### (1) General information

NMR spectra were recorded with a device, Bruker AVANCE III HD 400 (400 MHz for ¹H, 100 MHz for ¹³C, 373 Hz for ¹⁹F), in a solvent shown. Chemical shifts were reported in millions (ppm) relative to the signal of internal tetramethylsilane (δ 0 ppm for ¹H) in a CDCl₃ solution. NMR spectral data were reported as chloroform-d (7.26 ppm for ¹H), DMSO-d₆ (2.50 ppm for ¹H), CD₃OD-d₄ (3.31 ppm for ¹H), CD₃CN-d₄ (3.31 ppm for ¹H), or chloroform-d (77.16 ppm for ¹³C), C₆H₅CF₃ (-63.24 ppm for ¹⁹F). Multiplicities were reported using the following abbreviations: s, singlet; d, doublet; t, triplet; q, quartet; m, multiplet; br, broad; and J, Hertz coupling constant.

IR spectra were recorded on a Perkin Elmer Spectrum One FT-IR spectrophotometer. Only the most intense and/or structurally important absorptions were reported as IR data in cm⁻¹.

All reactions were monitored by thin-layer chromatography. Thin-layer chromatography was performed on a 0.2 mm E. Merck silica gel plate (60F-254) with UV light and visualized with a p-anisaldehyde solution, cerium sulfate, a ninhydrin ethanol solution, I₂-SiO₂, or 10% ethanolic phosphomolybdic acid. Merck silica gel 60 (0.063 to 0.200 mm) was used for column chromatography.

ESI TOF mass spectra were measured with Waters LCT Premier (R) XE. HRMS (ESI-TOF) was calibrated with leucine enkephalin (SIGMA) as an internal standard.

Gel permeation chromatography (GPC) for quantitative analysis was performed using a refractive index detector, model RI-5, available from Japan Analytical Industry Co., Ltd., and a ultraviolet light detector, model 310, available from Japan Analytical Industry Co., Ltd., equipped with a polystyrene gel column (JAIGEL-1H,20 mm x 600 mm) using chloroform as a solvent (3.5 mL/min) by model LC 605 (recycle preparative HPLC), available from Japan Analytical Industry Co., Ltd.

Analytical HPLC was performed using an SSC-3461 pump equipped with an SSC-5410 UV detector and a Waters 2475 fluorescence detector.

Using the Glass Contour solvent purification system, dry THF, toluene, MeCN, and CH₂Cl₂ were obtained. DMF was dried over activated molecular sieves 4A.

### (2) Synthesis of (2,2-dimethyl-5-((naphthalene-2-ylmethoxy)methyl)-1,3-dioxane-5-yl)methanol (2-29)

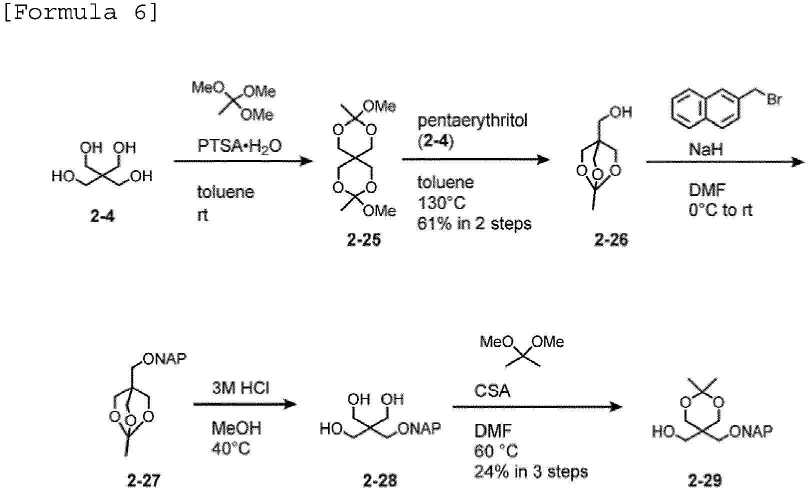

### (2-1) Synthesis of 4-(hydroxy methyl)-1-methyl-2,6,7-trioxabicyclo[2,2,2]-octane (2-26)

To a stirred solution of pentaerythritol (2-4) (10.3 g, 73.4 mmol, 1.00 eq.) in toluene (29.5 mL), were added p-toluenesulfonic acid monohydrate (130.2 mg, 0.734 mmol, 0.0100 eq.) and trimethyl orthoacetate (30.3 mL, 220 mmol, 3.00 eq.) at room temperature. After stirring at room temperature for 18 hours, the reaction mixture was evaporated in vacuo. The residue was used for the next reaction without further purification.

To a stirred solution of the residue in toluene (40.5 mL), was added pentaerythritol (2-4) (10.1 g, 73.4 mmol, 1.00 eq.) at room temperature. After stirring at 130°C for 11 hours, the reaction mixture was neutralized with NEt₃ and evaporated in vacuo. The residue was recrystallized from toluene/Et₂O to give 4-(hydroxy methyl)-1-methyl-2,6,7-trioxabicyclo[2,2,2]-octane (2-26) as white crystals (7.17 g, 44.8 mmol, 61%).
¹H NMR (400 MHz, CDCl₃) δ 4.04 (s, 6H, H-b), 3.47 (d, 2H, H-a, J = 3.2 Hz), 1.47 (s, 3H, H-c); ¹³C NMR (100 MHz, CDCl₃) δ 108.6, 69.4, 69.0, 61.3, 35.7, 23.5, 23.4; FT-IR (neat) 3648, 3006, 2952, 2882, 1716, 1541, 1507, 1158, 1038, 870 (cm⁻¹)

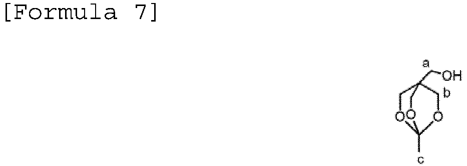

### References

### 2-26: O. Linnenberg, A. Kondinski, C. Stocker, K. Y. Monakhov* Dalton Trans., 2017, 46, 15636-15640

### (2-2) Synthesis of (2,2-dimethyl-5-((naphthalene-2-ylmethoxy)methyl)-1,3-dioxane-5-yl)methanol (2-29)

To a stirred solution of 60% sodium hydride (3.02 g, 74.9 mmol, 1.20 eq.) washed three times with dry hexane in DMF (16.5 mL), was added a solution of 4-(hydroxy methyl)-1-methyl-2,6,7-trioxabicyclo[2,2,2]-octane (2-26) (10.0 g, 62.4 mmol, 1.00 eq.) in DMF (34.2 mL) at 0°C. The reaction mixture was stirred at room temperature for 1 hour. Then, a solution of 2-(bromomethyl)naphthalene (13.8 g, 62.4 mmol, 1.00 eq.) in DMF (21.2 mL) was added to the reaction mixture at 0°C. After stirring at room temperature for 3 hours, EtOH and water were added to the stirred solution under cooling. The aqueous layer was extracted twice with EtOAc. The combined extracts were washed with brine, dried over MgSO₄, filtered, and concentrated in vacuo. The residue was used for the next reaction without further purification.

To the stirred solution of the residue in MeOH (100 mL), was added 3.0 M HCl (10.0 mL) at room temperature. After stirring at 40°C for 14 hours, the reaction mixture was evaporated in vacuo. The residue was used for the next reaction without further purification.

To the stirred solution of the residue in DMF (27.2 mL), were added CSA (15.6 mg, 0.065 mmol, 0.0012 eq.) and 2,2-dimethoxypropane (8.00 mL, 65.2 mmol, 1.20 eq.) at room temperature. After stirring at 60°C for 13 hours, the reaction mixture was neutralized with NEt₃ and poured into H₂O. The aqueous layer was extracted twice with ethyl acetate. The combined extracts were washed with brine, dried over MgSO₄, filtered, and concentrated in vacuo. The residue was purified by column chromatography on silica gel using hexane:ethyl acetate (50:50), followed by recrystallization from hexane/EtOAc, to obtain (2,2-dimethyl-5-((naphthalene-2-ylmethoxy)methyl)-1 3-dioxane-5-yl)methanol (2-29) (4.70 g, 14.9 mmol, 24%). ¹H NMR (400 MHz, CDCl₃) δ 7.86-7.80 (m, 3H, H-aromatic), 7.74 (s, 1H, H-aromatic), 7.50-7.40 (m 3H, H-aromatic), 4.68 (s, 2H, H-f), 3.75 (s, 2H, H-c), 3.74 (s, 2H, H-c), 3.69 (d, 2H, H-b, J = 5.8 Hz), 3.59 (s, 2H, H-e), 2.45 (t, 1H, H-a, J = 5.3 Hz), 1.41 (s, 3H, H-d), 1.39 (s, 3H, H-d);¹³C NMR (100 MHz, CDCl₃) δ 135.4, 133.3, 133.2, 128.5, 128.0, 127.8, 126.7, 126.3, 126.1, 125.6, 98.6, 74.0, 72.3, 65.1, 63.0, 39.1, 24.2, 23.5; FT-IR (neat) 3446, 2990, 2870, 1372, 1200, 1081, 1050, 827, 752, 420 (cm⁻¹)

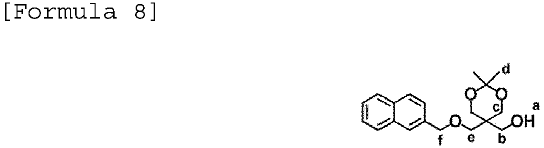

### (3) Synthesis of (2,2-dimethyl-5-((naphthalene-2-ylmethoxy)methyl)-1,3-dioxane-5-yl)methyl(4-methyl)benzene sulfonate (2-16) and 2,2-dimethyl-5-((naphthalene-2-ylmethoxy)methyl)-1,3-dioxane-5-yl)methyl trifluoromethanesulfonate (2-18)

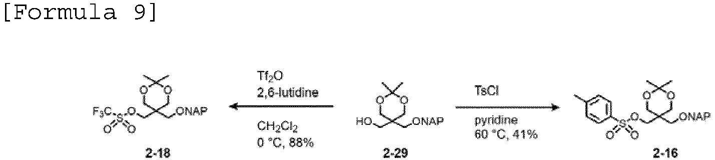

### (3-1) Synthesis of (2,2-dimethyl-5-((naphthalene-2-ylmethoxy)methyl)-1,3-dioxane-5-yl)methyl(4-methyl)benzene sulfonate (2-16)

To a stirred solution of (2,2-dimethyl-5-((naphthalene-2-ylmethoxy)methyl)-1,3-dioxane-5-yl)methanol (2-29) (435 mg, 1.39 mmol, 1.00 eq.) in pyridine (7.00 mL), was added p-toluenesulfonyl chloride (532 mg, 2.78 mmol, 2.00 eq.) at room temperature. After stirring at 75°C for 4.5 hours, the reaction mixture was poured into H₂O. The aqueous layer was extracted twice with EtOAc. The combined extracts were washed with brine, dried over MgSO₄, filtered, and concentrated in vacuo. The residue was purified by column chromatography on silica gel using hexane:ethyl acetate (80:20), to obtain (2,2-dimethyl-5-((naphthalene-2-ylmethoxy)methyl)-1,3-dioxane-5-yl)methyl(4-methyl)benzene sulfonate (2-16) (530 mg, 1.13 mmol, 41%).
¹H NMR (400 MHz, CDCl₃) δ 7.84 - 7.75 (m, 5H, aromatic), 7.66 (s, 1H, aromatic), 7.50-7.44 (m, 2H, aromatic) 7.34 (d, 1H, aromatic) 7.26-7.23 (m, 2H, aromatic) 4.55 (s, 2H, H-e) 4.17 (s, 2H, H-a), 3.73 (d, 2H, J = 12.0 Hz, H-c), 3.65 (d, 2H, H-c, J = 12.0 Hz), 3.40 (s, 2H, H-b), 2.32 (s, 3H, -PhCH₃), 1.35 (s, 3H, H-d), 1.28 (s, 3H, H-d); ¹³C NMR (100 MHz, CDCl₃) δ 144.9, 135.5, 133.4, 133.1, 132.7, 129.9, 128.3, 128.2, 128.0, 127.9, 126.3, 126.3, 126.1, 125.5, 98.7, 73.6, 69.4, 68.9, 62.3, 38.8, 25.2, 22.2, 21.7; FT-IR (neat) 2867, 1457, 1362, 1190, 1177, 1088, 977, 815, 666, 476 (cm⁻¹)

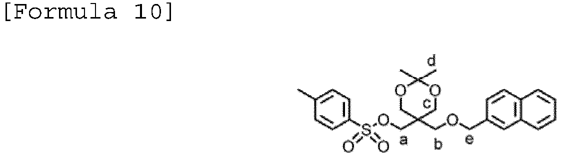

### (3-2) Synthesis of 2,2-dimethyl-5-((naphthalene-2-ylmethoxy)methyl)-1,3-dioxane-5-yl)methyl trifluoromethanesulfonate (2-18)

To a stirred solution of (2,2-dimethyl-5-((naphthalene-2-ylmethoxy)methyl)-1,3-dioxane-5-yl)methanol (2-29) (50.0 mg, 0.172 mmol, 1.00 eq.) in CH₂Cl₂ (0.860 mL), was added 2,6-lutidine (60.0 µL, 0.344 mmol, 2.00 eq.) and trifluoromethanesulfone acid anhydride (40.0 µL, 0.206 mmol, 1.20 eq.) at 0°C. After stirring at 0°C for 0.5 hours, the reaction mixture was poured into a saturated NH₄Cl aqueous solution. The aqueous layer was extracted twice with EtOAc. The combined extracts were washed with brine, dried over MgSO₄, filtered, and concentrated in vacuo. The residue was purified by column chromatography on silica gel using hexane:EtOAc(80:20), to obtain (2,2-dimethyl-5-((naphthalene-2-ylmethoxy)methyl)-1,3-dioxane-5-yl)methyl trifluoromethanesulfonate (2-18) (68.0 mg, 0.152 mmol, 88%) .
¹H NMR (400 MHz, CDCl₃) δ 7.85-7.81 (m, 3H, aromatic), 7.72 (s, 1H, aromatic), 7.51-7.39 (m, 3H, aromatic), 4.77 (s, 2H, H-a), 4.64 (s, 2H, H-e), 3.79 (d, 2H, J = 12.4 Hz, H-c), 3.71 (d, 2H, J = 12.4 Hz, H-c) 3.37 (s, 2H, H-b) 1.39 (s, 6H, H-d); ¹³C NMR (100 MHz, CDCl₃) δ 135.1, 133.3, 133.2, 128.5, 128.0, 127.9, 126.7, 126.4, 126.2, 125.7, 99.0, 75.8, 73.9, 68.4, 62.1, 39.2, 26.5, 20.9; ¹⁹F NMR (373 MHz, CDCl₃) δ -75.2; FT-IR (neat) 2993, 2872, 1415, 1374, 1246, 1203, 1146, 1087, 941, 824, 617, 477 (cm⁻¹)

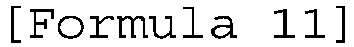

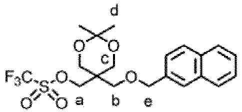

### (4) Synthesis of 5-iodomethyl-5-((naphthalene-2-ylmethoxy)methyl)-2,2,-dimethyl-1,3-dioxane (2-17)

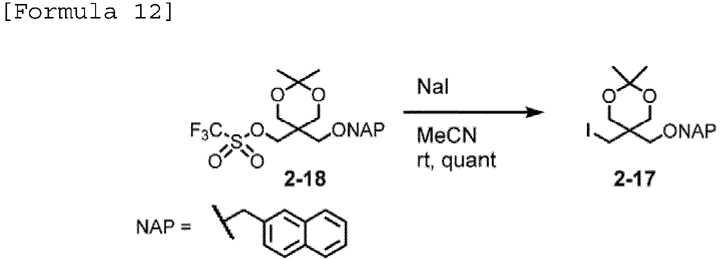

To a stirred solution of (2,2-dimethyl-5-((naphthalene-2-ylmethoxy)methyl)-1,3-dioxane-5-yl)methyl trifluoromethanesulfonate (2-18) (10.0 mg, 0.0223 mmol, 1.00 eq.) in CH₃CN (0.300 mL), was added sodium iodine (7.70 mg, 0.0514 mmol, 2.30 eq.) at room temperature. After stirring at room temperature for 1 hour, the reaction mixture was poured into H₂O. The aqueous layer was extracted twice with EtOAc. The combined extracts were washed with brine, dried over MgSO₄, filtered, and concentrated in vacuo. The residue was purified by short-path column chromatography on silica gel using EtOAc, to obtain 5-iodomethyl-5-((naphthalene-2-ylmethoxy)methyl)-2,2,-dimethyl-1,3-dioxane (2-17) (9.5 mg, 0.0223 mmol, quant.).
¹H NMR (400 MHz, CDCl₃) δ 7.85-7.83 (m, 3H, aromatic), 7.77 (s, 1H, aromatic), 7.50-7.45 (m, 3H, aromatic), 4.67 (s, 2H, H-e), 3.82 (d, 2H, H-c, J = 12.0 Hz), 3.72 (d, 2H, H-c, J = 12.0 Hz), 3.51 (s, 2H, H-a or H-b), 3.42 (s, 2H, H-a or H-b), 1.41 (s, 3H, H-d), 1.39 (s, 3H, H-d); ¹³C NMR (100 MHz, CDCl₃) δ 135.7, 133.4, 133.2, 128.3, 128.0, 127.8, 126.5, 126.3, 126.0, 125.8, 98.7, 73.8, 71.3, 65.0, 37.3, 29.8, 23.8, 23.7, 11.7; FT-IR (neat) 2923, 2863, 1717. 1505, 1370, 1270, 1092, 834, 742, 451 (cm⁻¹)

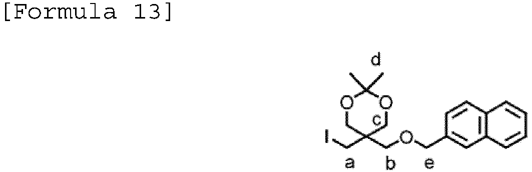

### [Example 4] Measurement 2 of generation rate of ²¹¹At-labeled compound

²¹¹At labeled metaastatobenzylguanidine (²¹¹At-MABG) was synthesized under the following two types of reaction conditions. An ²¹¹At solution dissolved in 90.9 µL of MeOH containing 0.5 mg of K₂CO₃ was put into a reaction container, a vent needle connected to activated carbon was attached, and the solvent was removed with a nitrogen gas. Conditions (1): 50 µL of trifluoroacetic acid (TFA) containing 0.4 mg of N-chlorosuccinimide (NCS) and 50 µL of TFA containing 0.1 mg of a labeling precursor, metatrimethylsilyl benzyl guanidine (MTMSBG) were added thereto, or conditions (2): TFA (50 µL) and TFA (50 µL) containing 0.1 mg of a precursor, MTMSBG, were added thereto. Then, a vent needle connected to activated carbon was attached after heating at 70°C for 10 minutes, and TFA was removed by spraying a nitrogen gas under heating at 70°C. The reaction product was dissolved in 0.5 mL of distilled water and passed through a Sep-Pak tC18 Light (duplicate) column preactivated with 3 mL of ethanol (EtOH) and 6 mL of water for injection. It was washed with 0.5 mL × 2 of water for injection, eluted with 0.5 mL × 4 of 5% EtOH, and further eluted with 1.0 mL × 2 of 5% EtOH, each fraction was collected in a vial, and radioactivity was measured (Table 3). HPLC was also used to measure the MABG purity of each fraction (Table 4).

MTMSBG was purchased from ABX, Prominence, available from SHIMADZU CORPORATION, was used for HPLC, SPD-M20A (available from SHIMADZU CORPORATION) was used for UV detection, and GABI STAR (Raytest) was used for radioactivity analysis. The analysis conditions were as follows. The elution time of ²¹¹At-MABG was 8.2 minutes.
Column: Triart C18 Plus 250 × 4.6 mm (YMC)
Mobile phase: MeCN-0.1%TFA (gradient program)
Time (min): 0 20 20.5 25.5
MeCN (%): 30 70 100 100
Flow rate: 1 mL/min
Column temperature: 25°C
Detector: UV detector (220 nm)

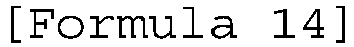

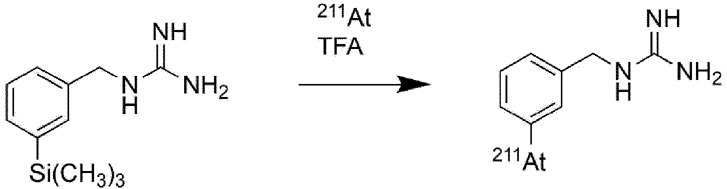

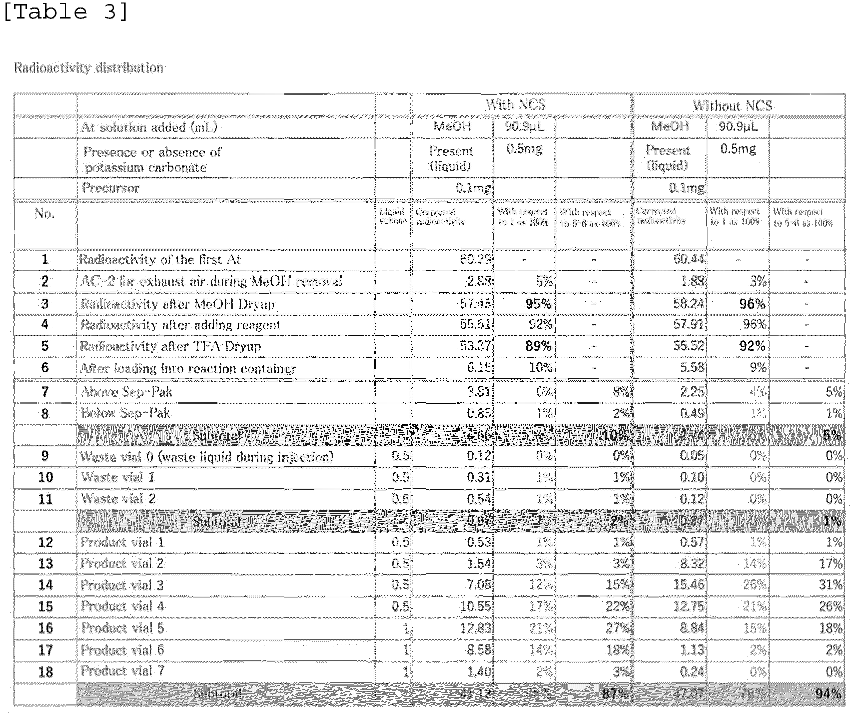

From Table 3, the radiochemical yield after concentration of ²¹¹At was 67.3% with NCS (collecting product vial 2-6) and 75.1% without NCS (collecting product vial 2-5). The labeling synthesis of ²¹¹At-MABG was result in good yields regardless of the presence or absence of the oxidant NCS.

**[Table 4]**

| HPLC analysis results | | | |
|---|---|---|---|
| With NCS | | | |
| | Elution volume | Proportion of radioactivity of each fraction relative to Sep-Pak injection volume | Purity of ²¹¹At-MABG (HPLC) |
| Waste vial 1 | 0.5 mL | 0.7% | 44.9% |
| Waste vial 2 | 0.5 mL | 1.1% | 75.0% |
| Product vial 1 | 0.5 mL | 1.1% | 63.5% |
| Product vial 2 | 0.5 mL | 3.3% | 98.8% |
| Product vial 3 | 0.5 mL | 15.0% | 98.8% |
| Product vial 4 | 0.5 mL | 22.3% | 98.4% |
| Product vial 5 | 1.0 mL | 27.2% | 98.6% |
| Product vial 6 | 1.0 mL | 18.2% | 94.2% |
| Product vial 7 | 1.0 mL | 3.0% | 85.5% |

| Without NCS | | | |
|---|---|---|---|
| | Elution volume | Proportion of radioactivity of each fraction relative to Sep-Pak injection volume | Purity of MABG (HPLC) |
| Waste vial 1 | 0.5 mL | 0.2% | 16.8% |
| Waste vial 2 | 0.5 mL | 0.2% | 22.0% |
| Product vial 1 | 0.5 mL | 1.1% | 77.3% |
| Product vial 2 | 0.5 mL | 16.7% | 99.0% |
| Product vial 3 | 0.5 mL | 31.0% | 97.3% |
| Product vial 4 | 0.5 mL | 25.5% | 95.6% |
| Product vial 5 | 1.0 mL | 17.7% | 89.0% |
| Product vial 6 | 1.0 mL | 2.3% | 65.9% |
| Product vial 7 | 1.0 mL | 0.5% | 38.0% |

From Table 4, the radiochemical purity was 97.7% with NCS (collecting product vial 2-6) and 95.5% without NCS (collecting product vial 2-5). The radiochemical purity of the ²¹¹At-MABG to be obtained was high regardless of the presence or absence of the oxidant NCS.

### [Industrial Applicability]

The present invention relates to a method for producing astatine-211, and therefore it can be used in industrial fields for producing pharmaceuticals or the like using astatine-211.

## Claims

1. A method for producing astatine-211, comprising the following steps (1) to (5):
(1) a step of generating astatine-211 by irradiating bismuth with α rays;
(2) a step of heating the astatine-211 generated in step (1) to vaporize;
(3) a step of cooling the astatine-211 that has been vaporized in step (2) and collecting the astatine-211 with a volatile and polar solvent to obtain an astatine-211 solution;
(4) a step of adding a weak acid salt to the astatine-211 solution obtained in step (3) to obtain an astatine-211 solution containing the weak acid salt; and
(5) a step of removing the solvent from the astatine-211 solution containing the weak acid salt obtained in step (4).

2. The method for producing astatine-211 according to claim 1, wherein the weak acid salt is a carbonate or bicarbonate.

3. The method for producing astatine-211 according to claim 1, wherein the weak acid salt is a carbonate of an alkali metal or a bicarbonate of an alkali metal.

4. The method for producing astatine-211 according to claim 1, wherein the weak acid salt is NaHCO₃, Na₂CO₃, KHCO₃, K₂CO₃, CsHCO₃, or Cs₂CO₃.

5. The method for producing astatine-211 according to any one of claims 1 to 4, wherein the volatile and polar solvent is methanol, ethanol, 2-propanol, or acetonitrile.

6. A method for producing an astatine-211-labeled compound, comprising reacting the astatine-211 produced by the method for producing astatine-211 according to any one of claims 1 to 5 with a labeling precursor compound to produce an astatine-211-labeled compound, wherein the reaction is a nucleophilic substitution reaction.

7. The method for producing an astatine-211-labeled compound according to claim 6, wherein the labeling precursor compound is a compound having an alkylsulfonyloxy group, a haloalkylsulfonyloxy group, or an arylsulfonyloxy group, and the astatine-211-labeled compound is a compound in which the alkylsulfonyloxy group, the haloalkylsulfonyloxy group, or the arylsulfonyloxy group in the labeling precursor compound is substituted with astatine-211.

8. The method for producing an astatine-211-labeled compound according to claim 6, wherein the labeling precursor compound is a compound having a p-toluenesulfonyloxy group or a trifluoromethanesulfonyloxy group, and the astatine-211-labeled compound is a compound in which the p-toluenesulfonyloxy group or the trifluoromethanesulfonyloxy group in the labeling precursor compound is substituted with astatine-211.

9. A method for producing an astatine-211-labeled compound, comprising reacting the astatine-211 produced by the method for producing astatine-211 according to any one of claims 1 to 5 with a labeling precursor compound to produce an astatine-211-labeled compound, wherein the reaction is an electrophilic substitution reaction.

10. The method for producing an astatine-211-labeled compound according to claim 9, wherein the electrophilic substitution reaction is an aromatic electrophilic substitution reaction.

11. The method for producing an astatine-211-labeled compound according to claim 9 or 10, wherein the labeling precursor compound is a compound having a trialkylsilyl group or a trialkylstannyl group, and the astatine-211-labeled compound is a compound in which the trialkylsilyl group or the trialkylstannyl group in the labeling precursor compound is substituted with astatine-211.

12. The method for producing an astatine-211-labeled compound according to claim 9 or 10, wherein the labeling precursor compound is a compound having a trimethylsilyl group or a tributylstannyl group, and the astatine-211-labeled compound is a compound in which the trimethylsilyl group or the tributylstannyl group in the labeling precursor compound is substituted with astatine-211.

## Patentansprüche

1. Verfahren zum Herstellen von Astatin-211, umfassend die folgenden Schritte (1) bis (5):
(1) einen Schritt des Erzeugens von Astatin-211 durch Bestrahlung von Wismut mit α-Strahlen;
(2) einen Schritt des Erhitzens des in Schritt (1) erzeugten Astatins-211 zum Verdampfen;
(3) einen Schritt des Abkühlens des in Schritt (2) verdampften Astatins-211 und des Sammelns des Astatins-211 mit einem flüchtigen und polaren Lösungsmittel, um eine Astatin-211-Lösung zu erhalten;
(4) einen Schritt des Hinzufügens eines schwachen Säuresalzes zu der in Schritt (3) erhaltenen Astatin-211-Lösung, um eine Astatin-211-Lösung zu erhalten, die das schwache Säuresalz enthält; und
(5) einen Schritt des Entfernens des Lösungsmittels aus der in Schritt (4) erhaltenen Astatin-211-Lösung, die das schwache Säuresalz enthält.

2. Verfahren zum Herstellen von Astatin-211 nach Anspruch 1, wobei das schwache Säuresalz ein Carbonat oder Bicarbonat ist.

3. Verfahren zum Herstellen von Astatin-211 nach Anspruch 1, wobei das schwache Säuresalz ein Carbonat eines Alkalimetalls oder ein Bicarbonat eines Alkalimetalls ist.

4. Verfahren zum Herstellen von Astatin-211 nach Anspruch 1, wobei das schwache Säuresalz NaHCO₃, Na₂CO₃, KHCO₃, K₂CO₃, CsHCO₃, oder Cs₂CO₃ ist.

5. Verfahren zum Herstellen von Astatin-211 nach einem der Ansprüche 1 bis 4, wobei das flüchtige und polare Lösungsmittel Methanol, Ethanol, 2-Propanol oder Acetonitril ist.

6. Verfahren zum Herstellen einer Astatin-211-markierten Verbindung, umfassend das Reagieren des Astatins-211, das durch das Verfahren zum Herstellen von Astatin-211 nach einem der Ansprüche 1 bis 5 hergestellt wurde, mit einer Markierungsvorläuferverbindung, um eine mit Astatin-211 markierte Verbindung herzustellen, wobei die Reaktion eine nukleophile Substitutionsreaktion ist.

7. Verfahren zum Herstellen einer mit Astatin-211 markierten Verbindung nach Anspruch 6, wobei die Markierungsvorläuferverbindung eine Verbindung mit einer Alkylsulfonyloxygruppe, einer Haloalkylsulfonyloxygruppe oder eine Arylsulfonyloxygruppe ist, und die mit Astatin-211 markierte Verbindung eine Verbindung ist, in der die Alkylsulfonyloxygruppe, die Haloalkylsulfonyloxygruppe oder die Arylsulfonyloxygruppe in der Markierungsvorläuferverbindung mit Astatin-211 substituiert ist.

8. Verfahren zum Herstellen einer mit Astatin-211 markierten Verbindung nach Anspruch 6, wobei die Markierungsvorläuferverbindung eine Verbindung mit einer p-Toluolsulfonyloxygruppe oder einer Trifluormethansulfonyloxygruppe ist, und die Astatin-211-markierte Verbindung eine Verbindung ist, in der die p-Toluolsulfonyloxygruppe oder die Trifluormethansulfonyloxygruppe in der Markierungsvorläuferverbindung mit Astatin-211 substituiert ist.

9. Verfahren zum Herstellen einer mit Astatin-211 markierten Verbindung, umfassend das Reagieren des Astatins-211, das nach dem Verfahren zum Herstellen von Astatin-211 nach einem der Ansprüche 1 bis 5 hergestellt wurde, mit einer Markierungsvorläuferverbindung, um eine mit Astatin-211 markierte Verbindung herzustellen, wobei die Reaktion eine elektrophile Substitutionsreaktion ist.

10. Verfahren zum Herstellen einer Astatin-211-markierten Verbindung nach Anspruch 9, wobei die elektrophile Substitutionsreaktion eine aromatische elektrophile Substitutionsreaktion ist.

11. Verfahren zum Herstellen einer mit Astatin-211 markierten Verbindung nach Anspruch 9 oder 10, wobei die Markierungsvorläuferverbindung eine Verbindung mit einer Trialkylsilylgruppe oder einer Trialkylstannylgruppe ist, und die mit Astatin-211 markierte Verbindung eine Verbindung ist, in der die Trialkylsilylgruppe oder die Trialkylstannylgruppe in der Markierungsvorläuferverbindung mit Astatin-211 substituiert ist.

12. Verfahren zum Herstellen einer mit Astatin-211 markierten Verbindung nach Anspruch 9 oder 10, wobei die Markierungsvorläuferverbindung eine Verbindung mit einer Trimethylsilylgruppe oder einer Tributylstannylgruppe ist, und die mit Astatin-211 markierte Verbindung eine Verbindung ist, in der die Trimethylsilylgruppe oder die Tributylstannylgruppe in der Markierungsvorläuferverbindung mit Astatin-211 substituiert ist.

## Revendications

1. Procédé de production d'astate-211, comprenant les étapes (1) à (5) suivantes :
(1) une étape consistant à générer de l'astate-211 en irradiant du bismuth avec des rayons α ;
(2) une étape consistant à chauffer l'astate-211 généré à l'étape (1) pour le vaporiser ;
(3) une étape consistant à refroidir l'astate-211 qui a été vaporisé à l'étape (2) et à collecter l'astate-211 avec un solvant volatil et polaire pour obtenir une solution d'astate-211 ;
(4) une étape consistant à ajouter un sel d'acide faible à la solution d'astate-211 obtenue à l'étape (3) pour obtenir une solution d'astate-211 contenant le sel d'acide faible ; et
(5) une étape consistant à éliminer le solvant de la solution d'astate-211 contenant le sel d'acide faible obtenue à l'étape (4).

2. Procédé de production d'astate-211 selon la revendication 1, dans lequel le sel d'acide faible est un carbonate ou un bicarbonate.

3. Procédé de production d'astate-211 selon la revendication 1, dans lequel le sel d'acide faible est un carbonate d'un métal alcalin ou un bicarbonate d'un métal alcalin.

4. Procédé de production d'astate-211 selon la revendication 1, dans lequel le sel d'acide faible est NaHCO₃, Na₂CO₃, KHCO₃, K₂CO₃, CsHCO₃, ou Cs₂CO₃.

5. Procédé de production d'astate-211 selon l'une quelconque des revendications 1 à 4, dans lequel le solvant volatil et polaire est le méthanol, l'éthanol, le 2-propanol, ou l'acétonitrile.

6. Procédé de production d'un composé marqué à l'astate-211, comprenant la réaction de l'astate-211 produit par le procédé de production d'astate-211 selon l'une quelconque des revendications 1 à 5 avec un composé précurseur de marquage pour produire un composé marqué à l'astate-211, dans lequel la réaction est une réaction de substitution nucléophile.

7. Procédé de production d'un composé marqué à l'astate-211 selon la revendication 6, dans lequel le composé précurseur de marquage est un composé présentant un groupe alkylsulfonyloxy, un groupe halogénoalkylsulfonyloxy, ou un groupe arylsulfonyloxy, et le composé marqué à l'astate-211est un composé dans lequel le groupe alkylsulfonyloxy, le groupe halogénoalkylsulfonyloxy, ou le groupe arylsulfonyloxy dans le composé précurseur de marquage est substitué par l'astate-211.

8. Procédé de production d'un composé marqué à l'astate-211 selon la revendication 6, dans lequel le composé précurseur de marquage est un composé présentant un groupe p-toluènesulfonyloxy ou un groupe trifluorométhanesulfonyloxy, et le composé marqué à l'astate-211 est un composé dans lequel le groupe p-toluènesulfonyloxy ou le groupe trifluorométhanesulfonyloxy dans le composé précurseur de marquage est substitué par l'astate-211.

9. Procédé de production d'un composé marqué à l'astate-211, comprenant la réaction de l'astate-211 produit par le procédé de production d'astate-211 selon l'une quelconque des revendications 1 à 5 avec un composé précurseur de marquage pour produire un composé marqué à l'astate-211, dans lequel la réaction est une réaction de substitution électrophile.

10. Procédé de production d'un composé marqué à l'astate-211 selon la revendication 9, dans lequel la réaction de substitution électrophile est une réaction de substitution électrophile aromatique.

11. Procédé de production d'un composé marqué à l'astate-211 selon la revendication 9 ou 10, dans lequel le composé précurseur de marquage est un composé présentant un groupe trialkylsilyle ou un groupe trialkylstannyle, et le composé marqué à l'astate-211 est un composé dans lequel le groupe trialkylsilyle ou le groupe trialkylstannyle dans le composé précurseur de marquage est substitué par l'astate-211.

12. Procédé de production d'un composé marqué à l'astate-211 selon la revendication 9 ou 10, dans lequel le composé précurseur de marquage est un composé présentant un groupe triméthylsilyle ou un groupe tributylstannyle, et le composé marqué à l'astate-211 est un composé dans lequel le groupe triméthylsilyle ou le groupe tributylstannyle dans le composé précurseur de marquage est substitué par l'astate-211.
